# EUROPEAN PATENT SPECIFICATION

(11) **EP 1 051 191 B1**
(45) Date of publication and mention of the grant of the patent: **20.09.2006**
(21) Application number: 98930436.5
(22) Date of filing: 19.06.1998
(51) Int. Cl.: A61K 39/395

(54) **CD154 BLOCKADE THERAPY FOR PANCREATIC ISLET TISSUE TRANSPLANTATION in primates**
CD154-BLOCKADETHERAPIE DER PANKREASINSELZELLTRANSPLANTATION bei Primaten
THERAPIE DE BLOCAGE PAR CD154 POUR TRANSPLANTATION DE TISSUS D'ILOTS PANCREATIQUES chez les primates

(30) Priority: 20.06.1997 US 50267 P; 09.03.1998 US 77265 P
(43) Date of publication of application: 15.11.2000
(73) Proprietor: Biogen Idec MA Inc., Cambridge, Massachusetts 02142 (US); University of Miami, Miami, FL 33136 (US)
(72) Inventor: KENYON, Norma, S., Miami, FL 33158 (US); RICORDI, Camillo, Miami Beach, FL 33158 (US); THOMAS, David, W., Wellesley, MA 02181 (US); BURKLY, Linda, West Newton, MA 02165 (US)
(74) Representative: UEXKÜLL & STOLBERG
(86) International application number: PCT/US1998/012892
(87) International publication number: WO 1998/058669

(56) References cited:
- WO-A-95/28957
- BUHLMANN J E ET AL: "Therapeutic potential for blockade of the CD40 ligand, gp39." JOURNAL OF CLINICAL IMMUNOLOGY, (1996 MAR) 16 (2) 83-9. REF: 56 JOURNAL CODE: HRC. ISSN: 0271-9142., XP002079820 United States
- ROSSINI A A ET AL: "Induction of immunological tolerance to islet allografts." CELL TRANSPLANTATION, (1996 JAN-FEB) 5 (1) 49-52. JOURNAL CODE: B02. ISSN: 0963-6897., XP002079821 United States
- INVERARDI L ET AL: "Transplantation of pancreas and islets of Langerhans: a review of progress" IMMUNOLOGY TODAY, vol. 17, no. 1, January 1996, page 7-9 XP004034634
- ZHENG X X ET AL: "Blockade of CD40L -CD40 costimulatory pathway in a DST pre-sensitization model leads to a state of allo-Ag specific anergy and permits prolong islet allograft survival." 30TH ANNUAL MEETING OF THE AMERICAN SOCIETY OF NEPHROLOGY, SAN ANTONIO, TEXAS, USA, NOVEMBER 2-5, 1997. JOURNAL OF THE AMERICAN SOCIETY OF NEPHROLOGY 9 (PROGRAM AND ABSTR. ISSUE). 1997. 670A. ABSTRACT A3124. ISSN: 1046-6673, XP002079822
- KIRK A D ET AL: "CTLA4-Ig and anti-CD40 ligand prevent renal allograft rejection in primates." PROCEEDINGS OF THE NATIONAL ACADEMY OF SCIENCES OF THE UNITED STATES OF AMERICA 94 (16). 1997. 8789-8794. ISSN: 0027-8424, XP002079823
- FOSTER D W: "DIABETES MELLITUS." WILSON, J. D., ET AL. (ED.). HARRISON'S PRINCIPLES OF INTERNAL MEDICINE, TWELFTH EDITION, VOLS. 1 AND 2. XXX+1026P.(VOL. 1);XXX+1183P.(VOL. 2) MCGRAW-HILL, INC.: NEW YORK, NEW YORK, USA; LONDON, ENGLAND, UK. ILLUS. MAPS. (1991) 0 (0), 1739-1759. ISBN, XP002094491

## Description

### Field of the invention

The invention relates generally to the suppression of unwanted immune responses, particularly of counter-adaptive T-lymphocyte mediated immune responses. The invention relates in particular to the prevention, treatment, suppression or reversal of immune-system driven rejection of grafted tissue or a grafted organ in a recipient host.

### Background of the Invention

Organ transplantation between genetically non-identical individuals invariably results in immunological rejection of the organ through T cell dependent mechanisms, unless the rejection process is bridled by administering drugs that suppress T cell function. Several U.S. Patents disclose the use of such immunosuppressant drugs for inhibiting graft rejection, including U.S. Nos. 5,104,858; 5,008,246; and, 5,068,323. Other conventional agents are described in Suthanthiran et al. (1994), 331 New Eng. Med. J. 365-376. Both calcineurin phosphatase inhibitors and glucocorticosteroids are used clinically, and both prevent the T cell mediated release of activating cytokines, particularly IL-2. However, therapy with these types of conventional agents remains imperfect. Both types act by impairing signalling through the T cell antigen receptor (TCR), the sole mediator of T cell antigen specificity, and act on all T cells indiscriminately. In addition, the effect of these drugs is not lasting, such that cessation of treatment generally results in graft loss. Thus, in order to maintain viable, functional integration of the graft, transplant recipients must suffer the consequences of long-term, non-specific immunosuppresion. These consequences include an increased risk of infection and malignancy, as well as toxicity, particularly to sensitive organs or tissues, such as the kidney, liver and pancreas.

Islet cell transplantation (ICT) can result in the reversal of hyperglycemia and normalization of metabolic control of blood glucose (Ricordi, Diabetes Reviews 4:356-369, 1996; Scharp et al., Diabetes 39:515-518, 1990; Socci et al., Acta Diabetot 28:151-157, 1991; Warnock et al, Diabetologia 34:55-58, 1991; Ricordi et al., Transplantation 53:407-414, 1992; Gores et al., Lancet 341:19-21, 1993; Alejandro et al., Diabetes 46:1983-1989, 1997; in individuals afflicted with diabetes mellitus (DM). Even in the absence of insulin independence, administration of reduced dosages of exogenous insulin in transplant recipients with functioning islet allografts (basal c-peptide production > 1.0 ng/mL) results in excellent metabolic control and normalization of hemoglobin Alc (HbAIc) (Ricordi. Diabetes Reviews 4:356-369, 1996; Scharp et al., Diabetes 39:515-518, 1990; Socci et al., Acta Diabetot 28:151-157, 1991; Warnock et al., Diabetologia 34:55-58, 1991; Ricordi et al., Transplantation 53:407-414, 1992; Gores et al., Lancet 341:19-21, 1993; Alejandro et al., Diabetes 46:1983-1989, 1997). Hypoglycemia has not been observed, and functioning islet allografts in recipients with autoimmune diabetes have now been documented at over 6 years post-transplant (Alejandro et al., Diabetes 46:1983-1989, 1997). Despite these significant advances, broad based application of islet cell transplantation to control DM has been limited by the requirement for chronic, generalized immunosuppression of the recipient. This limitation is related not only to the risks associated with chronic immunosuppression, but also to the diabetogenic effects of the immunosuppressive drugs currently used.

These limitations of current therapies to control DM have stimulated widespread interest in developing therapies for the induction of donor-specific immunological tolerance, thus obviating the need for life-long immunosuppression of graft recipients. Promising initial results have been obtained by several investigators, using a variety of rodent model systems for allotransplantation. When tested in large-animal preclinical models (e.g., canine, non-human primate), however, the rodent results have been found to be poorly predictive of the results of ICT in models more closely mimetic of human ICT.

There is accordingly a need for improved or more effective immunosuppressive or immunomodulatory treatments for graft recipients, including humans. In particular, there is a need for treatments that do not require pan-T cell immunosuppression, i.e., treatments that do not leave the recipient vulnerable to malignancies or opportunistic infection. More pointedly, there is a need for treatments that have lesser toxicity than currently available therapeutic agents. Similarly, there is a need for treatments that promote lasting functional integration of the graft, i.e., integration that persists beyond termination of the course of treatment.

### Summary of the Invention

It is an object of this invention to provide a CD40:CD 154 binding interruptor, which is an anti-CD40L or anti-CD154 monoclonal antibody or a Fab⁻, F(ab')₂⁻ fragment, or single-chain antibody thereof such as a CD154 blocking agent, for use in therapy, particularly for use in therapy to mitigate, delay or reverse immunological rejection of grafted tissue. A more general object of the invention is to improve the availability of tissue grafts, particularly insulin-producing tissue grafts, by providing immunomodulatory compositions that allow functional integration of non-autologous tissue (e.g., allogeneic or xenogeneic tissue) into a recipient host. A further general object is to prevent, mitigate, attenuate or treat diabetes mellitus (DM).

The present invention rests on the discovery that use of a CD40:CD154 binding interruptor, such as a CD 154 blocking agent, whether used alone or in combination with another therapeutic agent, such as an immunomodulatory agent or a tolerizing agent, attenuates, suppresses, prevents, delays or reverses counter-adaptive immune system rejection of grafted insulin-producing tissue in a primate recipient host, without the need for pan-suppression of the recipient's immune system.
The present invention relates to the use of a CD40:CD154 binding interruptor in the manufacture of a medicament for inhibiting rejection, prolonging survival, reversing rejection, or preserving function of an insulin-producing tissue graft in a human graft recipient, or for restoring metabolic control of glucose metabolism in a human graft recipient of an insulin-producing tissue graft, wherein an effective amount of the CD40:CD154 binding interruptor is administered to the graft recipient and wherein the CD40:CD154 binding interruptor is an anti-CD40L (anti-CD154) monoclonal antibody, an Fab fragment of an anti-CD40L (anti-CD154) monoclonal antibody, an F(ab')₂ fragment of an anti-CD40L (anti-CD154) monoclonal antibody or a single chain anti-CD40L (anti-CD154) monoclonal antibody.

An exemplary CD 154 blocking agent is a monoclonal antibody (MAb), particularly one having the antigen-specific binding characteristics of the 5c8 MAb produced by the hybridoma deposited under ATCC Accession No. HB 10916, as disclosed in U.S. Patent 5,474,771.
The foregoing uses can be practiced with all types of insulin-producing tissue grafts, such as whole pancreatic tissue or pancreatic islets isolated by conventional techniques. Thus, the invention is suitable for use where the graft recipient (recipient host) is a mammal, preferably a primate, most preferably a human. In particular, the invention is suitable for use where the graft recipient is afflicted with, or at risk of, an impairment of metabolic control of glucose metabolism, such as DM. The graft donor can be a non-syngeneic member of the same phylogenetic species as the graft recipient (i.e., an allogeneic donor, providing allograft tissue), or a member of a distinct phylogenetic species (i.e., a xenogeneic donor, providing xenograft tissue). If a xenogeneic donor is used as the graft tissue source, preferably the donor is relatively MHC-compatible with the recipient host; for example, a baboon or chimpanzee would be preferred as a donor for grafting tissue into a human. The invention can be used to promote engraftment of other types of insulin-producing tissue, including cell populations of isolated adult or fetal islet β cells, or cultured islet β cells (whether derived from a primary cell culture or an immortalized cell line). Indeed, the invention can be used to promote engraftment of any cell inducibly or stably expressing an insulin gene, such as an engineered or host cell produced by conventional genetic engineering techniques. Optionally, the insulin-producing tissue can be physically separated from tissues of the recipient by an immunoisolation device.
In view of the foregoing it should be clear that the invention comprises the use of a CD40:CD154 binding interruptor in the manufacture of a medicament for restoring metabolic control of glucose metabolism in a primate in need thereof. This method involves implanting insulin producing tissue into the primate; and treating the primate with a CD40:CD154 binding interruptor, preferably with a CD154 blocking agent. In preferred embodiments, the CD154 blocking agent is a monoclonal antibody having the antigen-specific binding properties of MAb 5c8. In an exemplary protocol, which has been validated by testing in relevant large-animal preclinical models of human DM, engraftment is induced by administration of the MAb prior to ICT, followed-(preferably) by at least two administrations of the MAb within a two-week period following ICT (i.e., following implantation of insulin-producing tissue). Thereafter, as desired, engraftment is maintained by administration of the MAb one month (defined as four weeks) after ICT. The maintenance can be repeated as necessary or as deemed prudent.

Optionally, engraftment can be enhanced by concurrently treating the primate with a tolerizing agent, by which is meant any agent that preserves engraftment beyond the cessation of immunomodulatory or immunosuppressive therapy. An exemplary tolerizing agent comprises bone marrow tissue, or a population of bone marrow derived cells, that are MHC-compatible with the tissue graft (here, with the insulin-producing tissue). Preferably, the bone marrow or cells are syngeneic with the donor or source of insulin-producing tissue. Long-term survival of the tolerizing agent in the graft recipient accordingly renders the recipient immunologically chimeric, a state manifested by donor-specific immunological tolerance. Populations of marrow-derived, CD34(+) hematopoeitic cells (stem cells) are particularly preferred as tolerizing agents. Especially preferred are populations of stem cells having a CD40(-) cell surface phenotype.

### Brief Description of the Drawings

The foregoing and other objects, features and advantages of the present invention, as well as the invention itself, will be more fully understood from the following description of preferred embodiments, when read together with the accompanying drawings, in which:
FIGURE 1 is a line graph which plots fasting blood glucose (FG) levels of baboon recipients of CD 154 blocking agent therapy for ICT as a function of post-operative day (POD).
FIGURE 2 is a line graph which plots FG levels of rhesus monkey recipients of CD 154 blocking agent therapy for ICT as a function of post-operative day (POD).
FIGURE 3 is a line graph which compares the rhesus monkey FG levels shown in FIGURE 2 with FG levels of a human afflicted with DM and receiving conventional insulin replacement therapy.

### Detailed Description of the Invention

T cell activation, and immunological processes dependent thereon, requires both T cell receptor (TCR) mediated signals and simultaneously delivered costimulatory signals. An important costimulatory signal is delivered by the ligation of CD40 on an antigen-presenting cell, such as a B cell, by CD40L (CD154) on a T cell. Human CD40 is a 50 kD cell surface protein expressed on mature B cells, as well as on macrophages and activated endothelial cells. CD40 belongs to a class of receptors involved in programmed cell death, including Fas/CD95 and the tumor necrosis factor (TNF) alpha receptor. Human CD154 (CD40L) is a 32 kD type II membrane glycoprotein with homology to TNF alpha that is transiently expressed, transiently, primarily on activated T cells. CD40:CD154 binding has been shown to be required for all T cell-dependent antibody responses. In particular, CD40:CD154 binding provides anti-apoptotic and/or lymphokine stimulatory signals.

The importance of CD40:CD154 binding in promoting T cell dependent biological responses was more fully appreciated when it was discovered that X-linked hyper-IgM syndrome (X-HIGM) in humans is the phenotype resulting from genetic lack of functional CD154. Affected individuals have normal or high IgM levels, but fail to produce IgG, IgA or IgE antibodies, and suffer from recurrent, sometimes severe, bacterial and parasitic infections, as well as an increased incidence of lymphomas and abdominal cancers. A similar phenotype is observed in non-human animals rendered nullizygous for the gene encoding CD154 (knockout animals). B cells of CD154 nullizygotes can produce IgM in the absence of CD40L:CD154 binding, but are unable to undergo isotype switching, or to survive normally after affinity maturation. Histologically, lymph node germinal centers fail to develop properly, and memory B cells arc absent or poorly developed. Functionally, these defects contribute to a severe reduction or absence of a secondary (mature) antibody response. Defects in cellular immunity are also observed, manifested by an increased incidence of bacterial and parasitic infections. Many of these cell-mediated defects are reversible by administration of IL-12 or IFN-gamma. These observations substantiate the view that normal CD40:CD154 binding promotes the development of Type I T-helper cell immunological responses.

A number of preclinical studies have established that agents capable of interrupting CD40:CD154 binding have promise as immunomodulating agents. In particular, studies involving small-animal organ or tissue transplantation models have shown that CD40:CD154 interruptors promote survival of allogeneic grafts. In selected models, transient administration of agents interfering with T cell costimulation has resulted in the induction of indefinite graft acceptance. Interruption of CD40:CD154 binding in particular has yielded promising results, since it appears that engagement of this counter-receptor pair precedes other costimulatory signals in chronology and hierarchy (Ranheim et al., J. Exp Med 177:925-935, 1993; Roy et al., Eur J Immunol 25:596-603, 1995; Han et al., J Inununol 155:556-567, 1995; Shinde et al., J Immunol 157:2764-2768, 1996; Yang et al., Science 273:1862-1864, 1996; Grewal et al., Science 273:1864-1867-1996; Lederman et al., J Immunol 149:3817-3826, 1992.). Blockade of CD40:CD154 binding has resulted in prolongation of cardiac (Larsen et al., Transplantation 61:4-9, 1996; Larsen et al., Nature 381:434438, 1996), cutaneous (Larsen et al., Nature 381:434438, 1996; Markees et al., Transplantation 64:329-335, 1997) and islet allografts (Parker et al., Proc Natl Acad Sci USA 92:9560-9564, 1995; Rossini et al., Cell Transplant 5:49-52) in rodents, and of allogeneic kidneys in primates (Kirk et al., Proc Natl Acad Sci USA 194:8789-8794, 1997). It has also been demonstrated to delay onset of autoimmune diabetes in non-obese diabetic (NOD) mice (Balasa et al., J Immunol 159:4620-4627, 1997). Lastly, it has been reported that interference with CD40:CD 154 binding prevents the production of inflammatory cytokines (Dechanet et al., J Immunol 159:5640-5647, 1997; Kiener et al., J. Inununol 155:4917-4925, 1995.

CD40:CD154 blockade thus may provide potentially powerful therapies for prevention of islet allograft or xenograft failures in individuals having defective glucose metabolism, such as Type I diabetes. However, as noted above, studies in rodent model systems have correlated poorly with the outcome of testing or therapy of large animals, including humans.

Disclosed herein are studies assessing the effects of a preferred CD154 blocking agent, a humanized MAb having the antigen-specific binding properties of MAb 5c8 (Lederman et al., J. Exp. Med. 175:10911101,1992), in large-animal preclinical models of islet allotransplantation. Specifically, the present models involve CD154 blockade monotherapy of baboons (Papio hamadryas) and other non-human primates. Results obtained from these studies strongly suggest that CD154 blockade monotherapy will promote long-term engraftment of insulin-producing tissue in humans, particularly humans afflicted with DM or a similar defect in glucose homeostasis.

The following discussion illustrates and exemplifies the variety of contexts and circumstances in which the invention can be practiced, as well as providing proof-of-principle studies involving specific embodiments of the invention.

### Recipient Hosts

The invention can be used for treatment or prophylaxis of any primate recipient of an insulin-producing tissue graft, or any primate in need of an insulin-producing tissue graft. Recipient hosts (also referred to as recipients or hosts) accordingly are afflicted with, or at risk of, a defect in metabolic control of blood glucose metabolism (glucose homeostasis). For example, the recipient can be hyper- or hypo-glycemic. The invention is particularly suitable for use with diabetic recipients, particularly recipients afflicted with diabetes mellitus (DM). The recipient is a primate, more preferably a higher primate, most preferably a human.

### Donor or Graft Tissue

The invention can be used with any type of insulin-producing tissue transplant or graft procedure, particularly procedures wherein the donor (graft) tissue is affected by, or at risk of, failure or rejection by the recipient host's immune system. In particular, the invention can be used in any context wherein the donor tissue is not histocompatible (MHC-compatible) with the recipient host. Thus, in addition to autologous or syngeneic donor tissue, the invention can be used with allogeneic or even xenogeneic donor tissue. The donor tissue can be derived, by conventional means, from a volunteer or other living donor, or from a cadaveric donor. Preferably, the donor is as histocompatible as practicable with the recipient host. Thus, where the recipient host is a human, autologous and allogeneic donor tissue is preferred. However, the donor tissue can be obtained from a heterologous species (in which case it is referred to as a heterograft), such as a non-human primate (e.g., a chimpanzee or a baboon), or another relatively compatible mammal (e.g., a pig).

In some embodiments, the donor tissue comprises an intact pancreas. In other embodiments, the donor tissue comprises a part, portion or biopsy of a donor pancreas. The donor pancreas can be obtained from a living donor or can be retrieved from a suitable cadaver. If a cadaveric donor is used, the pancreas is preferably exposed to cold ischemic conditions for no more than about eight hours. In still other embodiments, the donor tissue comprises insulin-producing cells, particularly isolated or suspended islets or islet cells, including cells withdrawn or excised from a fetal or adult donor, cells maintained in primary culture, or an immortalized cell line. Appropriate means for preparing donor islets or islet cell suspensions from whole pancreata are well known (see, e.g., Ricordi et al. (1988), 37 Diabetes 413-420; Tzakis et al. (1990), 336 Lancet 402-405; Linetsky et al. (1997), 46 Diabetes 1120-1123). Appropriate pancreata are obtained from donors essentially free of defects in blood glucose homeostasis. Other sources of insulin-producing cells include fetal islet progenitor cells, optionally expanded in primary culture. Any appropriate cell type can be used, however, including cells harboring exogenous genetic material encoding an expressible insulin gene. Thus, the invention encompasses the use of transfected or transformed host cells, which have been (or are derived from ancestor cells which have been) engineered to express insulin, either constitutively or inducibly (e.g., under control of a glucose-responsive promoter or enhancer). In other embodiments, the invention encompasses the use of pancreatic or other donor cell types derived from a transgenic mammal that has been engineered to include genetic material necessary for the production of insulin in some or all of its body tissues.

The insulin producing tissue (donor tissue) is introduced systemically or locally into the recipient host. For example, isolated, suspended or dispersed insulin-producing cells can be infused intravascularly, or implanted into a desired site, such as a bone marrow cavity, the liver, within the kidney capsule, intramuscularly, or intraperiotoneally. In some embodiments, the cells are mitotically competent and produce new tissue of donor origin. In other embodiments, the cells are not mitotically competent, but remain viable in the donor, and produce or express insulin. In any event, an effective amount of insulin-producing cells or tissue is implanted, by which is meant an amount sufficient to attenuate (detectably mitigate) the recipient's defect in glucose metabolism (e.g., hypoglycemia or hyperglycemia). Optimally, the amount is sufficient to restore the recipient's ability to maintain glucose homeostasis - that is, to free the recipient from dependence on conventional (e.g., injected or inhaled) insulin replacement therapy.

In some embodiments, the insulin-producing tissue is physically separated (isolated) from surrounding tissues of the recipient by an immunoisolation device. Appropriate devices protect the insulin-producing tissue from most effectors of cellular and humoral immunity, including but not limited to, leukocytes, immunoglobulin and complement. Thus, the immunoisolation device generally provides a semipermeable barrier, such as a membrane, having a pore size sufficient to prevent diffusion therethrough of molecules more massive than about 50 to 100 kD. The barrier defines an isolation chamber in which the insulin-producing tissue is disposed, and is free of any sites at which the insulin-producing tissue can physically contact cells or tissues external to the barrier. Any conventional device, envelope, capsule or microcapsule can be used, including single- or double-walled alginate microcapsules (e.g., as described in U.S. Pat. 5,227,298). Other conventional microcapsules include alginate polylysine microcapsules, chemically cross-linked alginate microcapsules, and capsules formed of other biocompatible polymers, formed into a structurally sound immunoisolation device of any desired shape or size (see, e.g., Jaink et al. (1996), 61 Transplantation 4).

In further embodiments, a tolerizing agent, such as bone marrow or cells derived therefrom, also is implanted into the recipient host. Any tolerogenic tissue or cell type can be used as a tolerizing agent, including pancreatic stromal cells as well as bone marrow cells. The tolerogenic cells are MHC-compatible with the insulin-producing tissue, and are preferably obtained from the donor who provided the insulin-producing tissue or are syngeneic therewith. Appropriate means for preparing bone marrow or cell populations thereof are well known (see, e.g., Sharp et al. (1984), 69 J. Immunol. Meth. 187-195, Fontes et al. (1995), in Methods in Cell Transplantation, Ricordi, ed., R.G. Landes Co., pub., pp. 619-628). Preferably, the bone marrow is processed using Ceprate® SC Stem Cell Concentration System (CellPro, Inc., Bothell, WA; see CellPro Investigator Brochure, rev. 06.01.97), or equivalent thereto, to provide a bone marrow derived cell population enriched in CD34(+) hematopoeitic cells. Standard fluorescence-activated cell sorting (FACS) analysis or immunofluorescence staining reveals that this population of CD34(+) cells is essentially free of CD40(+) cells, however some dim staining for CD40 may be observed. As the CD34(+) cell population is a dynamic stem cell population, it is believed that the presence of a low level of CD40(+) cells corresponds to the frequency at which the stem cells commence differentiation along a B cell lineage. Indeed, preliminary FACS studies have established that the only CD40(+) cells (which typically represent no more than about 0.7% sof the total) in the CD34(+) stem cell population also are CD19(+). CD19 is the earliest currently known B cell lineage marker. Accordingly, to ensure use of a true stem cell population as the tolerizing agent, the CD34(+) cells can be further depleted of CD40(+) cells by conventional negative selection means (e.g., selective cytolysis, cell sorting, panning).

### Exemplary CD40:CD154 Binding Interrupters

Therapeutic compounds useful for practice of the invention include any compound that blocks the interaction of cell surface CD40 (e.g., on B cells) with CD40L (CD 154) expressed, e.g., on the surface of activated T cells. CD40:CD154 binding interrupter compounds, such as CD154 blocking agents, that are specifically contemplated include polyclonal antibodies and monoclonal antibodies (MAbs), as well as antibody derivatives such as chimeric molecules, humanized molecules, molecules with reduced effector functions, bispecific molecules, and conjugates of antibodies. In a preferred embodiment, the antibody has the antigen-specific binding characteristics of MAb 5c8 produced by the hybridoma deposited under ATCC Accession No. HB 10916, as described in U.S. Patent 5,474,771. In a currently highly preferred embodiment, the antibody is a humanized 5c8. Other known antibodies against CD154 include antibodies ImxM90, ImxM91 and ImxM92 (obtained from Immunex), an anti-CD40L mAb commercially available from Ancell (clone 24-31, catalog # 353-020, Bayport, MN), and an anti-CD40L mAb commercially available from Genzyme (Cambridge, MA, catalog # 80-3703-01). Also commercially available is an anti-CD40L mAb from PharMingen (San Diego, catalog #33580D). Numerous additional anti-CD40L antibodies have been produced and characterized (see, e.g., WO 96/23071 of Bristol-Myers Squibb).

The invention also includes use of CD154 blocking agents of other types, such as complete Fab fragments, F(ab')₂ compounds, V_{H} regions, Fv regions, single chain antibodies (see, e.g., WO 96/23071), polypeptides, fusion constructs of polypeptides, fusions of CD40 (such as CD40Ig, as in Hollenbaugh et al., J. Immunol. Meth. 188:1-7, 1995), and small molecule compounds such as small semi-peptidic compounds or non-peptide compounds, all capable of blocking or interrupting CD40:CD154 binding. Procedures for designing, screening and optimizing small molecules are provided in PCT/US96/10664, filed June 21, 1996.

Various forms of antibodies also can be produced using standard recombinant DNA techniques (Winter and Milstein, Nature 349: 293-99, 1991). For example, "chimeric" antibodies may be constructed, in which the antigen binding domain from an animal antibody is linked to a human constant domain (an antibody derived initially from a nonhuman mammal in which recombinant DNA technology has been used to replace all or part of the hinge and constant regions of the heavy chain and/or the constant region of the light chain, with corresponding regions from a human immunoglobin light chain or heavy chain) (see, e.g., Cabilly et al., United States Pat. No. 4,816,567; Morrison et al., Proc. Natl. Acad. Sci. 81: 6851-55, 1984). Chimeric antibodies reduce the immunogenic responses elicited by animal antibodies when used for human therapy or prophylaxis.

In addition, recombinant "humanized" antibodies can be synthesized. Humanized antibodies are antibodies initially derived from a nonhuman mammal in which recombinant DNA technology has been used to substitute some or all of the amino acids not required for antigen binding with amino acids from corresponding regions of a human immunoglobin light or heavy chain. That is, they are chimeras comprising mostly human immunoglobulin sequences into which the regions responsible for specific antigen-binding have been inserted (see, e.g., PCT patent application WO 94/04679). Animals are immunized with the desired antigen, the corresponding antibodies are isolated and the portion of the variable region sequences responsible for specific antigen binding are removed. The animal-derived antigen binding regions are then cloned into the appropriate position of the human antibody genes in which the antigen binding regions have been deleted. Humanized antibodies minimize the use of heterologous (inter-species) sequences in antibodies for use in human therapies, and are less likely to elicit unwanted immune responses. Primatized antibodies can be produced similarly.

Another embodiment of the invention includes the use of human antibodies, which can be produced in nonhuman animals, such as transgenic animals harboring one or more human immunoglobulin transgenes. Such animals may be used as a source for splenocytes for producing hybridomas, as described in U.S. 5,569,825.

Antibody fragments and univalent antibodies also can be used in practice of this invention. Univalent antibodies comprise a heavy chain/light chain dimer bound to the Fc (or stem) region of a second heavy chain. "Fab region" refers to those portions of the chains which are roughly equivalent, or analogous, to the sequences which comprise the Y branch portions of the heavy chain and to the light chain in its entirety, and which collectively (in aggregates) have been shown to exhibit antibody activity. A Fab protein includes aggregates of one heavy and one light chain (commonly known as Fab'), as well as tetramers which correspond to the two branch segments of the antibody Y, (commonly known as F(ab)₂), whether any of the above are covalently or non-covalently aggregated, so long as the aggregation is capable of selectively reacting with a particular antigen or antigen family.

In addition, standard recombinant DNA techniques can be used to alter the binding affinities of recombinant antibodies with their antigens by altering amino acid residues in the vicinity of the antigen binding sites. The antigen binding affinity of a humanized antibody may be increased by mutagenesis based on molecular modeling (Queen et al., Proc. Natl. Acad. Sci. 86:10029-33, 1989; PCT patent application WO 94/04679). It may be desirable to increase or to decrease the affinity of the antibodies for CD40L, depending on the targeted tissue type or the particular treatment schedule envisioned. This may be done utilizing phage display technology (see, e.g., Winter et al., Ann. Rev. Immunol. 12:433-455, 1994; and Schier et al., J. Mol. Biol. 255:28-43, 1996). For example, it may be advantageous to treat a patient with constant levels of antibodies with reduced affinity for CD40L for semi-prophylactic treatments. Likewise, antibodies with increased affinity for CD40L may be advantageous for short-term treatments.

### Routes of Administration

The CD40:CD154 binding interruptors, including CD154 blocking agents, used in the invention can be administered in any manner which is medically acceptable. Depending on the specific circumstances, local or systemic administration may be desirable. Preferably, the agent is administered via a parenteral route such as by an intravenous, intraarterial, subcutaneous, intramuscular, intraorbital, intraventricular, intraperitoneal, subcapsular, intracranial, intraspinal, or intranasal injection, infusion or inhalation. The agent also can be administered by implantation of an infusion pump, or a biocompatible or bioerodable sustained release implant, into the recipient host, either before or after implantation of donor tissue. Alternatively, certain compounds of the invention, or formulations thereof, may be appropriate for oral or enteral administration. Still other compounds of the invention will be suitable for topical administration.

In further embodiments, the CD40:CD154 binding interruptor is provided indirectly to the recipient, by administration of a vector or other expressible genetic material encoding the interruptor. The genetic material is internalized and expressed in cells or tissue of the recipient, thereby producing the interruptor in situ. For example, a suitable nucleic acid construct would comprise sequence encoding one or more of the MAb 5c8 immunoglobulin (Ig) chains as disclosed in U.S. Pat. 5,474,771. Other suitable constructs would comprise sequences encoding chimeric or humanized versions of the MAb 5c8 Ig chains or antigen-binding fragments thereof. Still other suitable constructs would comprise sequences encoding part or all of other CD154-specific MAbs. The construct is delivered systemically or locally, e.g., to a site vicinal to the site of implantation of insulin-expressing tissue.

Alternatively, the vector or other genetic material encoding the interruptor is internalized within a suitable population of isolated cells to produce interuptor-producing host cells. These host cells then are implanted or infused into the recipient, either locally or systemically, to provide in situ production of the CD40:CD154 binding interruptor. Appropriate host cells include cultured cells, such as immortalized cells, as well as cells obtained from the recipient (e.g., peripheral blood or lymph node cells, such as natural killer (NK) cells).

In general, compounds of the invention are administered to the recipient host. However, the compounds also can be administered to the donor, or to the donor tissue. For example, a compound of the invention can be included in a perfusion or preservative fluid in which the donor tissue is stored or transported prior to its integration into the recipient host.

### Formulation

In general, the compound(s) used in practice of the invention are suspended, dissolved or dispersed in a pharmaceutically acceptable carrier or excipient. The resulting therapeutic composition does not adversely affect the recipient's homeostasis, particularly electrolyte balance. Thus, an exemplary carrier comprises normal physiologic saline (0.15M NaCl, pH 7.0 to 7.4). Other acceptable carriers are well known in the art and are described, for example, in Remington's Pharmaceutical Sciences, Gennaro, ed., Mack Publishing Co., 1990. Acceptable carriers can include biocompatible, inert or bioabsorbable salts, buffering agents, oligo- or polysaccharides, polymers, viscosity-improving agents, preservatives.

Any CD40:CD 154 binding interruptor, such as a CD 154 blocking agent, that is used in practice of the invention is formulated to deliver a pharmaceutically-effective or therapeutically-effective amount or dose, which is an amount sufficient to produce a detectable, preferably medically beneficial effect on the recipient. Medically beneficial effects would include preventing, delaying or attenuating deterioration of, or detectably improving, the recipient's medical condition. As an example, renal function and health of a kidney allograft or xenograft can be monitored by routinely measuring the concentrations of blood urea nitrogen or creatinine, or the volume or solute contents of urine, or the rate of clearance of relevant solutes from the blood into the urine. Similarly, glucoregulatory function and health of insulin-producing allograft or xenograft can be monitored by routinely measuring the concentrations of blood or urine glucose, glucose metabolites, or insulin, or measuring insulin response to glucose challenge, e.g., in a conventional glucose tolerance test. Thus, an effective amount of a therapeutic compound of the invention, such as a CD154 blocking agent, is any amount which detectably decreases the recipient's dependence on insulin replacement therapy. An optimal effective amount is one which substantially frees the recipient of dependence on exogenous insulin. More specifically, an effective amount is one which induces partial or substantially complete engraftment (acceptance and function) of donor insulin-producing tissue.

### Dosages and Frequency of Treatment

The amount of and frequency of dosing for any particular compound to be used in practice of the invention is within the skills and clinical judgement of ordinary practitioners of the tissue transplant arts, such as transplant surgeons. The general dosage and administration regime is established by preclinical and clinical trials, which involve extensive but routine studies to determine effective, e.g., optimal, administration parameters for the desired compound. Even after such recommendations are made, the practitioner will often vary these dosages for different recipient hosts based on a variety of considerations, such as the recipient's age, medical status, weight, sex, and concurrent treatment with other pharmaceuticals. Determining effective dosage and administration regime for each CD40:CD154 binding interruptor used to inhibit graft rejection is a routine matter for those of skill in the pharmaceutical and medical arts. The dosage amount and timecourse of should be sufficient to produce a clinically beneficial change in one or more indicia of the recipient's health status. Exemplary timecourse and dosage regimes are set forth in the proof-of-principle studies included herein. Essentially, the invention involves administration of a CD40:CD154 binding interruptor (exemplified by a humanized MAb 5c8, hu5c8) in an acceptance-inducing regime, followed if deemed prudent by an acceptance-maintaining regime.

To exemplify dosing considerations for an anti-CD40L compound, the following examples of administration strategies are given for an anti-CD40L mAb. The dosing amounts could easily be adjusted for other types of anti-CD40L compounds. In general, single dosages of between about 0.05 and about 50 mg/kg patient body weight are contemplated, with dosages most frequently in the 1-20 mg/kg range. For acute treatment, such as before or at the time of transplantation, or in response to any evidence that graft rejection is beginning, an effective dose of antibodies ranges from about 1 mg/kg body weight to about 20 mg/kg body weight, administered daily for a period of about 1 to 5 days, preferably by bolus intravenous administration. The same dosage and dosing schedule may be used in the load phase of a load-maintenance regimen, with the maintenance phase involving intravenous or intramuscular administration of antibodies in a range of about 0.1 mg/kg body weight to about 20 mg/kg body weight, for a treatment period of anywhere from weekly to 3 month intervals. Chronic treatment may also be carried out by a maintenance regimen, in which antibodies are administered by intravenous or intramuscular route, in a range of about 0.1 mg/kg body weight to about 20 mg/kg body weight, with interdose intervals ranging from about 1 week to about 3 months. In addition, chronic treatment may be effected by an intermittent bolus intravenous regimen, in which between about 1.0 mg/kg body weight and about 100 mg/kg body weight of antibodies are administered, with the interval between successive treatments being from 1 to 6 months. For all except the intermittent bolus regimen, administration may also be by oral, pulmonary, nasal or subcutaneous routes.

If desired, the effectiveness of the antibodies can be increased by administration serially or in combination with conventional anti-rejection therapeutic agents or drugs such as, for example, corticosteroids or immunosuppressants. Alternatively, the antibodies may be conjugated to a conventional agent. This advantageously permits the administration of the conventional agent in an amount less than the conventional dosage, for example, less than about 50% of the conventional dosage, when the agent is administered as monotherapy. Accordingly, the occurrence of many side effects associated with that agent should be avoided.

Combination therapies according to this invention for treatment of graft rejection include the use of anti-CD40L antibodies together with agents targeted at B cells, such as anti-CD 19, anti-CD28 or anti-CD20 antibody (unconjugated or radiolabeled), IL-14 antagonists, LJP394 (LaJolla Pharmaceuticals receptor blocker), IR-1116 (Takeda small molecule) and anti-Ig idiotype monoclonal antibodies. Alternatively, the combinations may include T cell/B cell targeted agents, such as CTLA4Ig, IL-2 antagonists, IL-4 antagonists, IL-6 antagonists, receptor antagonists, anti-CD80/CD86 monoclonal antibodies, TNF, LFA1/ICAM antagonists, VLA4/VCAM antagonists, brequinar and IL-2 toxin conjugates (e.g., DAB), prednisone, anti-CD3 MAb (OKT3), mycophenolate mofetil (MMF), cyclophosphamide, and other immunosuppressants such as calcineurin signal blockers, including without limitation, tacrolimus (FK506). Combinations may also include T cell targeted agents, such as CD4 antagonists, CD2 antagonists and IL-12.

For maintenance of graft integration, or in a period following suppression of an acute episode of graft rejection, a maintenance dose of anti-CD40L antibodies, alone or in combination with a conventional anti-rejection agent is administered, if necessary. Subsequently, the dosage or the frequency of administration, or both, may be reduced. Where no sign of graft rejection is evident, treatment might cease, with vigilant monitoring for signs of graft rejection. In other instances, as determined by the ordinarily skilled practitioner, occasional treatment might be administered, for example at intervals of four weeks or more. Recipient hosts may, however, require intermittent treatment on a long-term basis upon any recurrence of disease symptoms.

### Pre-Clinical Model Systems for Evaluating CD40:CD154 Interruptor Treatment Regimes

Preferred, exemplary model systems for testing efficacy of a CD40:CD154 interrupting compound (e.g., an anti-CD40L compound or a CD154 blocking agent, such as a MAb having the specificity of MAb 5c8) are the primate (baboon and/or rhesus monky) islet allograft models. Such primate models have been shown to be rigorous tests of immune manipulation: they are exquisitely sensitive to even minor changes in allograft function or adverse effects on recipient wound healing and immune system function. In additon, the models have obivous biological similarity to human renal transplantation. Specifically, genes that encode MHC proteins are well conserved between humans and the primates used as basis for the models, and the primates' rejection of vascularized organs closely parallels that seen in clinical settings.

### Baboon Model of ICT for pancreatectomy-induced diabetes.

Identification of donor-recipient pairs. Peripheral blood mononuclear cells (PBMC) from 10 potential recipients (Baboons, *Papio hamadryas,* male and female, 1-2 years of age, approximately 4.0 kg) from the Mannheimer facility (Homestead, FL) were used as responders against PBMC from 11 male donors (over 2 years of age, purchased from the Southwest Foundation in Texas) in a one way mixed leukocyte culture (MLC). The baboon MLC was performed via standardized methods for human MLC. With respect to low background and high specific reactivity, utilization of medium supplemented with human serum yielded superior results, as compared to media with baboon or fetal calf serum. The donors were of a sufficient size that enough islets and bone marrow were obtained from 1 donor to allow for transplantation into 2 recipients. In contrast to the modest MLC responses observed when PBMC from animals at Mannheimer were used as MLC responders and stimulators, the MLC reactivity between these animals was excellent, with all potential recipients yielding stimulation indices (S.I.) of ≥ 10.0 against the stimulators (donors, backgrounds < 200-300 counts per minute, cpm). An attempt was made to choose two recipients with similar MLC reactivity to the designated donor, and donor-recipient pairs with varying degrees of alloreactivity were chosen. An MLC S.I. of >10 was considered very reactive and was chosen as the minimal acceptable disparity; as a comparison, when animals from Mannheimer were used as donors and recipients, the MLC S.I, were usually less than 5.

Islet/bone marrow preparation and administration. Islets were separated from the pancreas one day prior to ICT (i.e., on study day -1) by minor modifications of the automated method for human islet isolation (Ricordi et al., *Diabetes* 37-. 413420, 1988; Selvaggi et al., *Transplant.Proc.* 29: 1967-1968, 1997) using Liberase® (0.47mg/ml collagenase solution, obtained from Boehringer-Mannheim, Indianapolis, IN). Cold ischemia time of the pancreata averaged 0.5 ± 0.1 hours. The islets were enriched in a three-layer discontinuous Euroficoll gradient (1.108, 1.096, 1.037), in which the digested pancreatic tissue was bottom-loaded with the 1.108 layer. A cell separator (COBE 299 1, COBE, Lakewood, CO) was used for centrifugation of the gradients (Robertson, Chadwick, Contractor, James, London. *Acta Diabetologica* 30: 93-98, 1993). The number, volume, and purity of islets obtained was determined as follows: the final islet preparation was suspended in 250 ml RPMI 1640 solution, three 100 ul samples were stained with dithizone (Latif et al., *Transplantation* 45: 827-830, 1988) and counted to assess total islet yield, and the data was mathematically converted to determine the total number of islets with an average-diameter of 150 pm (islet equivalent; IEQ) (Ricordi et al., *Acta Diabetol.Lat.* 27: 185-195, 1990).

For studies on enhancing tolerance, vertebral body was harvested from the pancreas donor and processed to obtain donor bone marrow cells (DBMC) generally according to routine modifications of methods for processing of human vertebral bodies. For recipients of donor bone marrow, infusions were administered on days 5 and II post ICT. A total dose of 10⁹ nucleated cells per kg recipient body weight was given.

Restraint of animals. For chemical restraint, ketamine hydrochloride was injected into the gluteus muscle (10 mg/kg of body weight). Prolongation of sedation was achieved by administering ketamine HCl, i.m., at a dose of 5 mg/kg. Additional ketamine was given whenever an animal responded to a toe-pinch stimulus. Since previous studies have shown that ketamine reduces first phase insulin response to glucose (FPIR), the ketamine dose was maintained as low as possible in all metabolic tests (Lehmann et al., *J.Med.Primatol.* 26: 312-321, 1997). The total dose of ketamine to maintain satisfactory sedation over a period of 30 minutes was 35 ± 2 mg/kg. Animals were physically restrained while sedated by ketamine. Surgical and vascular penetration sites were prepared using betadine and alcohol alternating scrubs. Indwelling catheters were placed intravenously and secured.

Pancreatectomy and ICT. On the day of ICT (study day 0), the islet preparation was centrifuged and the pellet was resuspended in supplemented CMRL 1066, followed by overnight culture at 22°C. Prior to transplantation, the preparation was centrifuged and the pellet was resuspended in 20 ml RPMI 1640 solution containing 2.5% donor serum and 200 IU heparin. The number of IEQ was determined immediately prior to transplantation. Total pancreatectomy was performed according to established surgical techniques. After completion of the total pancreatectomy, a 20G angiocatheter was inserted into one of the mesenteric vessel tributaries of the portal vein, and ICT was accomplished by gravity infusion of the islet preparation over a 10 min period.

Immunosuppression and post-operative care. FK506 (tacrolimus) was chosen as the immunosuppressant, as this drug is currently utilized in human ICT. FK506 administration commenced at 5 days prior to ICT. A dosage of 0.1 mg/kg/day, i.m. was administered to recipient baboons. Drug levels were monitored daily and dosage was adjusted to maintain trough levels of approximately 15 ng/ml. Humanized anti-CD154, (derived from MAb 5c8, Lederman et al., J. Exp. Med. 175:1091-1101,1992) was given i.v. on study days -1, 3, and 10 at a dosage of 10 or 20 mg/kg, and serum levels of 5c8 and anti-5c8 were assessed by ELISA.

For the first post-operative day (study day 1, or POD 1), the baboons were given intravenous fluids. The animals were subsequently fed a diet containing 60 g of carbohydrate per day, with 45 g of monkey biscuits (supplemented with viokase) and 15 g of fruit. Based on experience with the first two animals treated with anti-CD 154, subsequent baboons were treated with small subcutaneous insulin doses (approximately 0.5 U/kg of body weight per day), for a period of 14-20 days after islet transplantation, in order to prevent "exhaustion" of the islets, thus optimizing the conditions necessary for successful engraftment.

Monitoring. Fasting and post-prandial blood glucose (FG and PPG, respectively) were monitored via heel stick and blood testing with a Glucometer Elite, and at least once weekly, a blood sample was drawn to obtain plasma for testing of FG levels with a Beckman glucose analyzer. In general a blood sample was also obtained to confirm unusually high readings. Blood samples were drawn from all animals in the anti-CD154 study prior to each dose of MAb (prior to day -1 and just before antibody administration on days 3 and 10) and weekly thereafter. At approximately 3 months post-transplant, testing was decreased to every other week. The blood samples were used for phenotyping of peripheral blood to assess leukocyte subsets and determination of CBC and chemistries, 5c8 and anti-5c8 levels, insulin and C-peptide levels, and chimerism. Blood was drawn periodically for retesting of MLC reactivity to donor and 3rd party antigens.

Assays. Plasma insulin was assayed by a double antibody method (Linco Research, Inc., St. Charles, MO). The lower limit of detection was 20 pmol/l and the average intra-assay coefficient variation was 6%. C-peptide was assayed in plasma with a lower detection limit of 6% and and intra-assay coefficient of variation of 6%. Plasma glucose was measured using a glucose analyzer (Beckman Instruments, Palo Alto, CA). Whole blood capillary glucose levels were measured with an Elite Glucometer (Bayer, Elkhard, IN). Validity of the insulin assay for baboons was demonstrated by the parallelism of insulin concentrations in dilutions of serum with the insulin standard curve. Glucagon was measured using a double antibody assay (DPC, Los Angeles, CA). These commercial kits have been previously validated by serial dilutions (Goodner et al., *Diabetes* 38: 925-931, 1989).

Intravenous Glucose Tolerance Testing (IVGTT). It has been previously shown that *in vivo* islet-cell function tests give an accurate reflection of changes in β cell mass. (McCulloch et al., *Diabetes* 40: 673-679, 1991). Intravenous glucose tolerance tests were carried out after a 16- to 18-h overnight fast, as previously described (Lehmann et al., *J.Med.Primatol.* 26: 312-321, 1997). In brief, blood samples were collected at -10, -5, and 0 min. Then 0.5g glucose per kg of body weight in a 50% glucose solution was injected over 20 seconds into the saphenous vein. 1.5 ml samples were collected from the contralateral femoral artery at 1, 3, 5, 7, 10, 15, 20, 25, 30 min post injection. Thus, a total of 12 blood samples were withdrawn over a 40 min period. The samples were drawn into glass tubes containing 0.05ml 15% fluid EDTA and 0.2ml trasylol (500 K.I.U. aprotinin/ml blood), placed on ice, and centrifuged within 10 minn. Plasma was then frozen at -80°C and assayed later for glucose, immunoreactive insulin, and glucagon.

Statistical analysis and calculations. Results are set forth as means ± SEM. The glucose disappearance constant (Kg) was calculated from the NGTT as the slope of the decline of the logₑ (In) plasma glucose between 10 and 30 min. after the glucose injection, multiplied by 100. The acute insulin response to glucose (AIRG) was calculated as the incremental area under the insulin curve (AUC) between 1-10 min after the IV glucose injection. The incremental responses (AUCGlucose, AUCInsulin) were calculated by means of the trapezoidal rule with subtraction of the basal values from I to 30 minutes, Data were analyzed with Statistica for Windows software (Version 5.0, 1997, Statsoft, Inc., Tulsa. OK, USA).

### Results.

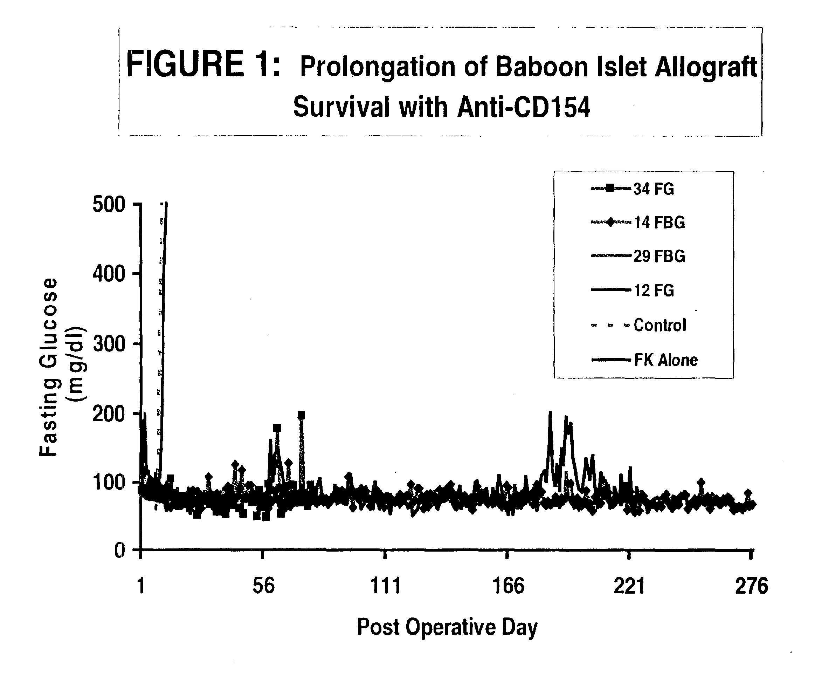

CD 154 blockade therapy prolongs survival and function of islet allografts. All baboons became normoglycemic immediately post transplant. As shown below in Table 1, ICT of allogeneic islets, in the absence of immunosuppressive or CD154 blockade therapy, resulted in rejection at day 8. Conventional immunosuppression with FK506 (alone or in combination with whole bone marrow or stem cell selected marrow) failed to improve islet survival, with animals rejecting at days 10, 8, and 10, respectively. In striking contrast, treatment of 4 out of 5 baboons with anti-CD154 (5C8) MAb resulted in extended islet allograft survival, well beyond that of control or FK506 treated animals. Results of this study also are set forth in line graph form in FIGURE 1, which plots fasting blood glucose (FG) as a function of POD. The present results demonstrate, for the first time, that CD154 blockade therapy prolongs acceptance of islet allografts in a nonhuman primate model of pancreatectomy-induced diabetes. Significantly, these results also demonstrate the ability of anti-CD154 therapy to reverse acute rejection.

CD 154 blockade therapy can be applied in conjunction with bone marrow cell transfer. Three baboons received delayed infusions of whole bone marrow (n=2) or stem cell selected marrow (selected over a Ceprate® column, CellPro, Bothell, WA) (n= 1) on PODs 5 and 11. These baboons were given 5C8 induction therapy (20 mg/kg days -1, 3, and 10) and then placed on monthly maintenance therapy, starting at POD 28. One animal did extremely well, remaining free of rejection until POD 241. A second animal experienced rejection on POD 112, was treated for rejection and maintained until POD 162. The third animal was treated for rejection on day 70 and maintained till POD 124.

Effect of CD154 blockade therapy on graft function, including control of glucose metabolism. Repeated IVGTTs in the control animals showed an excellent reproducibility of first phase insulin secretion (FPIS). In particular, one glucose tolerance test (IPGTT) and immunohistochemistry performed in an animal euthanized on day 79, revealed functioning graft tissue in the liver, with no residual insulin production from extrahepatic sites. Other animals in the study were found to have functioning islet allografts, with graft survivals ranging between >125 and >220 days. Repeated IVGTTs 4 to 16 weeks after pancreatectomy and islet transplantation showed almost identical Kg values in all animals, for up to 8 postoperative weeks. Kg values did decline thereafter in baboons treated with hu5c8 at the time of rejection. Stable values were observed in baboons treated with maintenance doses of hu5c8. Islet mass, as estimated by FPIS, was reduced with each rejection episode over time. In contrast, FPIS (follow-up 16 weeks) after islet transplantation for animals on hu5c8 maintenance therapy was well preserved. Two control animals were also studied. In some of the IVGTT, technical problems prompted administration of more ketamine than in the previous study, which resulted in a reduced Kg value and FPIR. However, on follow-up with the standard dose of ketamine, these indices were back to normal.

During the course of the present studies, it was discovered that graft rejection could be detected prior to elevation of FG by assessing the 2 hour PPG. Historically, islet graft rejection has been defined as two consecutive FGs greater than 250 mg/dl. It now has been discovered, however, that two consecutive 2 hour PPG greater than 150 mg/dl provides a sensitive index of the early stages of graft rejection. Application of antirejection therapy, whether with a CD154 blocker or a conventional antirejection agent, thus can be applied sufficiently early in the rejection process to permit rescue of metabolically viable graft tissue. For rescue of rejecting grafts by hu5c8, the same dosage regimen used to induce graft acceptance was repeated.

Conclusions from baboon studies. The foregoing studies demonstrate that hu5c8 promotes islet engraftment, allows long-term survival of allogeneic islets, and has no adverse effect on either insulin secretion or overall insulin sensitivity. Furthermore, these studies establish for the first time that engraftment can be maintained, and that reversal of islet rejection episodes in a primate model is possible, with CD154 blockade therapy. The therapies described herein thus can result in preservation insulin secretion and overall insulin sensitivity at pre-ICT levels in primate.

**Table 1. Prolongation of Non-Human Primate Allograft Survival with Anti-CD154**

| **Group** | **N** | **Species** | **Duration (in PODs) of Insulin Independence** |
|---|---|---|---|
| Control | 1 | Baboon | 8 |
| FK506 | 3 | Baboon | 8, 10, 10 |
| Anti-CD154 Induction + Anti-Rejection | 5 | Baboon | ^{a}8, ^{b}59, ^{c}229, ^{d}264, ^{e}284 |
| Anti-CD154 Induction + Maintenance | 2 | Baboon | ^{f}113, ^{g}238 |
| Anti-CD154 Induction + Maintenance | 4 | ^{h}Rhesus | ⁱ16, >80, >94, >166 |

| | | | |
|---|---|---|---|
| a) received a reduced dose of 5c8 b) Animal 34R; sacrificed on POD 79 with partial function, having experienced a rejection episode at POD 58, which was successfully reversed c) Animal 12R; sacrificed on POD 302 with partial function, having experienced six successfully treated episodes of rejection, beginning at POD 59 d) Animal 29R; sacrificed on POD 300, fully rejected, having experienced one episode of rejection e) Animal 14R; sacrificed on POD 301 with partial function, having experienced four successfully treated episodes of rejection, beginning on POD 31 f) sacrificed on POD 130, fully rejected g) sacrificed on POD 253 with partial function h) discussed below i) died insulin independent on POD 16 due to partial intestinal obstruction | | | |

### Rhesus Monkey Model of ICT for pancreatectomy-induced diabetes.

Unless specified, all procedures were generally as described above for the baboon model studies.

Animal procedures. SPF rhesus monkeys, 2-7 years of age, are readily available from COVANCE (Alice, TX) or the Mannheimer Foundation, Inc. (Homestead, FL) or similar vendors. Upon admission, all monkeys are examined to determine their general health, physical condition, and psychological status. All surgical procedures are carried under aseptic conditions. Animals are fasted for 12-18 hours before surgery and will be pre-anesthetized with i.m. ketamine (10 mg/kg) and atropine (0.04 mg/kg). Once the animal is sedated, an endotracheal tube and an i.v. catheter are promptly installed. The endotracheal tube is used to protect the airway and provide easy access for emergency drugs. The animals are anesthetized with an isoflurane and oxygen combination. Normal saline solution for injection is infused through the catheter throughout the entire ICT procedure at a rate of 10 ml/kg/hour.

A midline incision is made to gain access to the abdominal organs. For both donor and recipient animals, a total pancreatectomy is performed with preservation of the duodenum. Islets are isolated from the donor pancreas by conventional means for subsequent transplantation into pancreatectomized, diabetic monkeys. Following exsanguination of the donor under anesthesia, the vertebral bodies are removed through the abdominal incision with the aid of a Striker Saw. The bones are immediately processed to remove the marrow. In some recipients, bone marrow cells are infused into the recipient through the cephalic vein using a y-type blood set with filter.

After pancreatectomy of the recipient animals, a tributary of the inferior or superior mesentneric vein is catheterized and islets are infused via gravity drainage into the liver. Thereafter, surgical incisions are closed according to conventional surgical technique. At the end of the procedure, the animal are placed on oxygen alone and the endotracheal tube is removed when the animal sufficiently revived to control the airway. Post-ICT, recipients are placed in an ICU cage and observed until they are clinically stable. Antibiotics (Baytril) are given post-operatively (5 mg/kg i.m,, q 24 hours for 5 days). Bupomorphine (0.05 mg/kg, i.m.) is used as needed as an analgesic.

Monkeys are fasted after surgery and given gatorade p.o. on POD 1. On POD2, they are started on a twice daily soft diet, consisting of banana and softened (with water) high protein monkey chow containing viokase (I banana plus 4 biscuits). Normal diet is resumed on POD 3 (6-8 biscuits plus fruit, with viokase, twice daily). Each animal is fed individually to avoid competition during feeding. Sick monkeys are hand fed to improve genera! health and nutrition. Critically ill animals will be treated in isolation until they are well. Monkeys which are determined to be terminal or untreatable are sacrificed by fast i.v. injection of potassium chloride through an i..v. catheter.

Monitoring. Blood sampling for the purposes of glucose and insulin monitoring, immune monitoring, etc., does not exceed 1% of body weight at any one time or 7% of body weight over a one month period. Fasting blood glucose (FG) levels are determined by using a lancet to prick the heel, to obtain a small drop of blood for placement on a glucometer strip. In certain situations, e.g., where the glucose appears high (>200 mg/dl), venipuncture is performed to obtain a plasma sample for analysis on a Beckman glucose analyzer. Blood samples are also obtained prior to and at intervals post transplant to assess recipient anti-donor immunoreactivity. Intravenous glucose tolerance testing (IVGTT) is performed as described above, pre-transplant and at 4-6 week intervals thereafter.

### Results.

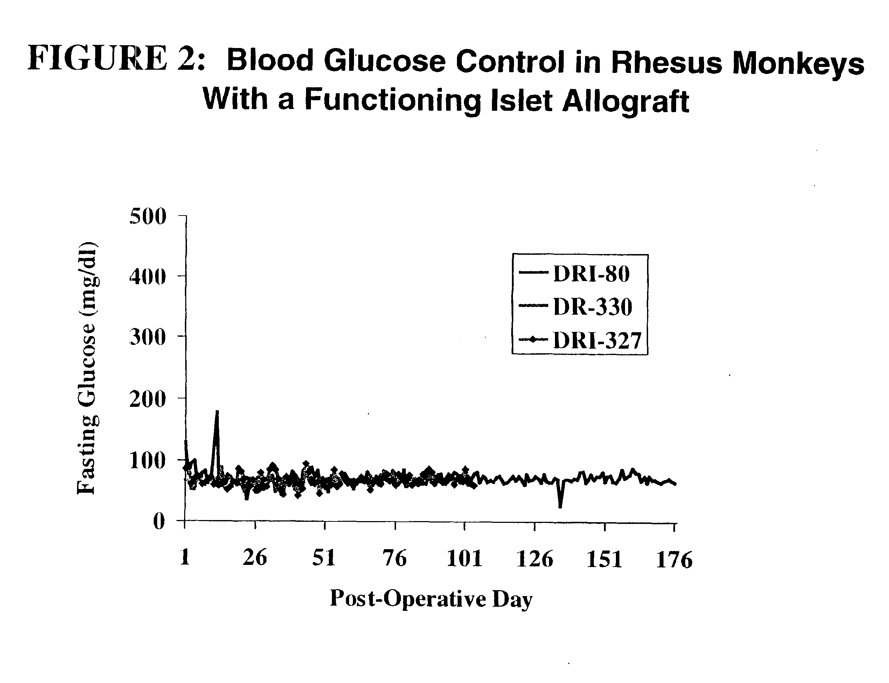

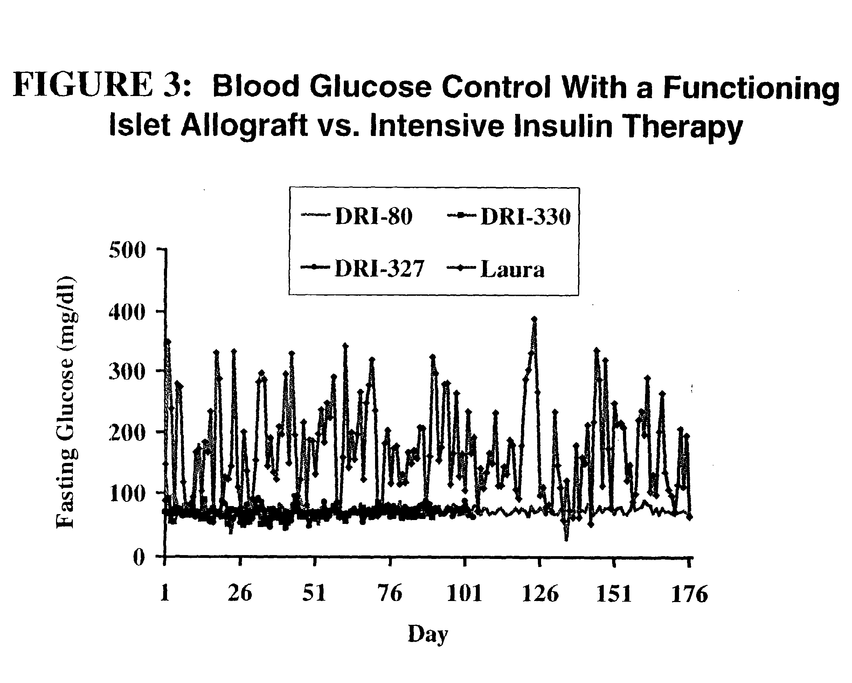

CD154 blockade therapy prolongs survival and function of islet allografts in diabetic rhesus monkeys, as well as in baboons. As shown above in Table 1, the present study demonstrated that hu5c8 monotherapy prolongs islet allograft acceptance in a second non-human primate species. This study utilized the acceptance-inducing regimen of hu5c8 administration on study days -1, 0, 3 and 10. A monthly acceptance-maintaining regimen also was followed, in order to maintain serum levels of hu5c8. The results, also set forth in FIGURE 2, are striking: functional islet allografts are maintained without the occurrence of rejection episodes. The significance of this finding is underscored by the comparative data set forth in FIGURE 3, which, in addition to the rhesus study animals, shows the FG plot of a human (designated LAURA) afflicted with DM and currently receiving intensive insulin replacement therapy. LAURA was diagnosed with DM at fourteen months of age, and at the time of the study, was six years of age and receiving insulin injections two to three times daily.

## Claims

1. Use of a CD40:CD154 binding interruptor in the manufacture of a medicament for inhibiting rejection, prolonging survival, reversing rejection, or preserving function of an insulin-producing tissue graft in a human graft recipient, or for restoring metabolic control of glucose metabolism in a human graft recipient of an insulin-producing tissue graft, wherein an effective amount of the CD40:CD154 binding interruptor is administered to the graft recipient and wherein the CD40:CD154 binding interruptor is an anti-CD40L (anti-CD154) monoclonal antibody, an Fab fragment of an anti-CD40L (anti-CD154) monoclonal antibody, an F(ab')₂ fragment of an anti-CD40L (anti-CD154) monoclonal antibody or a single chain anti-CD40L (anti-CD154) monoclonal antibody.

2. The use according to claim 1, wherein the anti-CD40L (anti-CD154) monoclonal antibody is:
(a) a monoclonal antibody having the antigen-specific binding characteristics of the 5c8 antibody produced by the hybridoma deposited under ATCC Accession No. HB 10916; or
(b) monoclonal antibody 5c8 produced by the hybridoma deposited under ATCC Accession No. HB 10916.

3. The use according to claim 1, wherein the anti-CD40L (anti-CD154) monoclonal antibody is selected from the group consisting of: chimeric antibodies, humanized antibodies and primatized antibodies.

4. The use according to any one of claims 1 to 3, wherein the insulin-producing tissue is:
(a) whole pancreatic tissue; or
(b) isolated pancreatic islets; or
(c) a cell population comprising isolated adult islet beta cells; or
(d) a cell population comprising isolated fetal islet beta cells; or
(e) a cell population comprising cultured islet beta cells; or
(f) a cell population comprising immortalized islet beta cells; or
(g) a cell population comprising host cells stably expressing an insulin gene; or
(h) a cell population comprising host cells inducibly expressing an insulin gene.

5. The use according to any one of claims 1 to 4, wherein the insulin-producing tissue is allogeneic or xenogeneic to the graft recipient.

6. The use according to any one of claims 1 to 5, wherein the graft recipient is afflicted with an impairment of metabolic control of glucose metabolism.

7. The use according to claim 6, wherein the graft recipient is afflicted with diabetes mellitus.

8. The use according to claim 1, wherein the medicament is for restoring metabolic control of glucose metabolism in a human graft recipient of an insulin-producing tissue graft, and said graft recipient is also a recipient of an effective amount of a tolerizing agent.

9. The use according to claim 8, wherein the tolerizing agent is bone marrow tissue that is MHC-compatible with the insulin-producing tissue.

10. The use according to claim 8, wherein the tolerizing agent is:
(a) bone marrow tissue that is syngeneic with the insulin-producing tissue; or
(b) whole bone marrow; or
(c) a population of CD34(+) hematopoietic cells; or
(d) a population of CD34(+) hematopoietic cells that are CD40(-).

11. The use according to claim 1, wherein the insulin-producing tissue in the graft recipient is physically separated from tissues of the recipient by an immunoisolation device.

12. The use according to claim 11, wherein the immunoisolation device:
(a) comprises a semipermeable barrier defining an isolated chamber in which the insulin-producing tissue is disposed; or
(b) is a capsule; or
(c) is a microcapsule.

## Patentansprüche

1. Verwendung eines Unterbrechers der CD40:CD154-Bindung zur Herstellung eines Medikaments zur Inhibierung der Abstoßung, zur Verlängerung des Überlebens, zur Umkehrung der Abstoßung oder zur Bewahrung der Funktion eines Insulin-produzierenden Gewebetransplantats bei einem menschlichen Transplantatempfänger oder zur Wiederherstellung der Stoffwechselkontrolle des Glukosestoffwechsels bei einem menschlichen Transplantatempfänger eines Insulin-produzierenden Gewebetransplantats, wobei eine wirksame Menge des Unterbrechers der CD40:CD154-Bindung dem Transplantatempfänger verabreicht wird, und wobei der Unterbrecher der CD40:CD154-Bindung ein monoklonaler Anti-CD40L (Anti-CD154)-Antikörper, ein Fab-Fragment eines monoklonalen Anti-CD40L (Anti-CD154)-Antikörpers, ein F(ab')₂-Fragment eines monoklonalen Anti-CD40L (Anti-CD154)-Antikörpers oder ein monoklonaler Einzelketten-Anti-CD40L (Anti-CD154)-Antikörper ist.

2. Verwendung nach Anspruch 1, **dadurch gekennzeichnet, dass** der monoklonale Anti-CD40L (Anti-CD154)-Antikörper
(a) ein monoklonaler Antikörper mit den Antigenspezifischen Bindungseigenschaften des Antikörpers 5c8 ist, der von dem Hybridom produziert wird, welches unter der ATCC-Zugangsnummer HB 10916 hinterlegt ist; oder
(b) der monoklonale Antikörper 5c8 ist, der von dem Hybridom produziert wird, welches unter der ATCC-Zugangsnummer HB 10916 hinterlegt ist.

3. Verwendung nach Anspruch 1, **dadurch gekennzeichnet, dass** der monoklonale Anti-CD40L (Anti-CD154)-Antikörper ausgewählt ist aus der Gruppe bestehend aus chimären Antikörpern, humanisierten Antikörpern und primatisierten Antikörpern.

4. Verwendung nach einem der Ansprüche 1 bis 3, **dadurch gekennzeichnet, dass** das Insulin-produzierende Gewebe
(a) Gesamt-Pankreasgewebe; oder
(b) isolierte Pankreas-Inseln; oder
(c) eine Zellpopulation, die isolierte adulte Beta-Inselzellen umfasst; oder
(d) eine Zellpopulation, die isolierte fetale Beta-Inselzellen umfasst; oder
(e) eine Zellpopulation, die kultivierte Beta-Inselzellen umfasst; oder
(f) eine Zellpopulation, die immortalisierte Beta-Inselzellen umfasst; oder
(g) eine Zellpopulation, die Wirtzellen umfasst, welche ein Insulin-Gen stabil exprimieren; oder
(h) eine Zellpopulation, die Wirtzellen umfasst, welche ein Insulin-Gen induzierbar exprimieren
ist.

5. Verwendung nach einem der Ansprüchen 1 bis 4, **dadurch gekennzeichnet, dass** das Insulin-produzierende Gewebe allogen oder xenogen in Bezug auf den Transplantatempfänger ist.

6. Verwendung nach einem der Ansprüche 1 bis 5, **dadurch gekennzeichnet, dass** der Transplantatempfänger an einer Störung der Stoffwechselkontrolle des Glukosestoffwechsels leidet.

7. Verwendung nach Anspruch 6, **dadurch gekennzeichnet, dass** der Transplantatempfänger an Diabetes mellitus leidet.

8. Verwendung nach Anspruch 1, **dadurch gekennzeichnet, dass** das Medikament zur Wiederherstellung der Stoffwechselkontrolle des Glukosestoffwechsels bei einem menschlichen Transplantatempfänger eines Insulin-produzierenden Gewebetransplantats ist, und der Transplantatempfänger auch ein Empfänger einer wirksamen Menge eines tolerisierenden Mittels ist.

9. Verwendung nach Anspruch 8, **dadurch gekennzeichnet, dass** das tolerisierende Mittel Knochenmarkgewebe ist, das mit dem Insulin-produzierenden Gewebe MHC-kompatibel ist.

10. Verwendung nach Anspruch 8, **dadurch gekennzeichnet, dass** das tolerisierende Mittel
(a) Knochenmarkgewebe, das syngen mit dem Insulin-produzierenden Gewebe ist; oder
(b) Gesamt-Knochenmark; oder
(c) eine Population von CD34(+) hämatopoetischen Zellen; oder
(d) eine Population von CD34(+) hämatopoetischen Zellen, die CD40(-) sind,
ist.

11. Verwendung nach Anspruch 1, **dadurch gekennzeichnet, dass** das Insulin-produzierende Gewebe in dem Transplantatempfänger von den Geweben des Empfängers physikalisch durch eine Immun-Isolierungsvorrichtung getrennt ist.

12. Verwendung nach Anspruch 11, **dadurch gekennzeichnet, dass** die Immun-Isolierungsvorrichtung
(a) eine semipermeable Barriere umfasst, die eine isolierte Kammer definiert, in der das Insulin-produzierende Gewebe gelagert ist; oder
(b) eine Kapsel ist; oder
(c) eine Mikrokapsel ist.

## Revendications

1. Utilisation d'un interrupteur de liaison CD40:CD154 dans la fabrication d'un médicament pour l'inhibition du rejet, la prolongation de la survie, l'inversion du rejet ou la préservation de la fonction d'une greffe de tissu produisant de l'insuline chez un receveur de greffe humain ou pour restaurer le contrôle métabolique du métabolisme du glucose chez un receveur de greffe humain d'une greffe d'un tissu produisant de l'insuline où une quantité effective d'un interrupteur de liaison CD40:CD154 est administrée au receveur de la greffe et/ou l'interrupteur de liaison CD40:CD154 est un anticorps monoclonal anti-CD40L (anti-CD154), un fragment Fab d'un anticorps monoclonal anti-CD40L (anti-CD154), un fragment F(ab')₂ d'un anticorps monoclonal anti-CD40L (anti-CD154) ou un anticorps monoclonal anti-CD40L (anti-CD154) monocaténaire.

2. Utilisation selon la revendication 1, où l'anticorps monoclonal anti-CD40L (anti-CD154) est:
(a) un anticorps monoclonal ayant les caractéristiques de liaison spécifique de l'antigène, de l'anticorps 5c8 produit par l'hybridome déposé sous le numéro d'accession ATCC HB 10916; ou
(b) l'anticorps monoclonal 5c8 produit par l'hybridome déposé sous le numéro d'accession ATCC HB10916.

3. Utilisation selon la revendication 1, où l'anticorps monoclonal anti-CD40L (anti-CD154) est sélectionné dans le groupe consistant en: anticorps chimériques, anticorps humanisés et anticorps primatisés.

4. Utilisation selon l'une quelconque des revendications 1 à 3, où le tissu produisant de l'insuline est:
(a) du tissu pancréatique entier; ou
(b) des îlots pancréatiques isolés; ou
(c) une population de cellules comprenant des cellules bêta d'îlots d'adulte isolés; ou
(d) une population de cellules comprenant des cellules bêta d'îlots foetaux isolés; ou
(e) une population de cellules comprenant des cellules bêta d'îlots cultivées; ou
(f) une population de cellules comprenant des cellules bêta d'îlots immortalisées; ou
(g) une population de cellules comprenant des cellules hôtes exprimant stablement un gène d'insuline; ou
(h) une population de cellules comprenant des cellules hôtes exprimant de manière inductible un gène d'insuline.

5. Utilisation selon l'une quelconque des revendications 1 à 4, où le tissu produisant de l'insuline est allogénéique ou xénogénéique vis-à-vis du receveur de la greffe.

6. Utilisation selon l'une quelconque des revendications 1 à 5, où le receveur de la greffe souffre d'une dégradation du contrôle métabolique du métabolisme du glucose.

7. Utilisation selon la revendication 6, où le receveur de la greffe est affligé de diabète sucré.

8. Utilisation selon la revendication 1, où le médicament est pour restaurer le contrôle métabolique du métabolisme du glucose chez un receveur de greffe humain d'une greffe de tissu produisant de l'insuline et ledit receveur de greffe est également un receveur d'une quantité efficace d'un agent de tolérisation.

9. Utilisation selon la revendication 8, où l'agent de tolérisation est un tissu de moelle osseuse qui est MHC compatible avec le tissu produisant l'insuline.

10. Utilisation selon la revendication 8, où l'agent de tolérisation est:
(a) du tissu de moelle osseuse qui est syngénéique avec le tissu produisant l'insuline; ou
(b) de la moelle osseuse entière; ou
(c) une population de cellules hématopoiétiques CD34(+); ou
(d) une population de cellules hématopoiétiques CD34(+) qui sont CD40(-).

11. Utilisation selon la revendication 1, où le tissu produisant de l'insuline chez le receveur de la greffe est physiquement séparé des tissus du receveur par un dispositif immunoisolant.

12. Utilisation selon la revendication 11, où le dispositif immunoisolant:
(a) comprend une barrière semiperméable définissant une chambre isolée dans laquelle le tissu produisant de l'insuline est disposé; ou
(b) est une capsule; ou
(c) est une microcapsule.
